# EUROPÄISCHE PATENTSCHRIFT

(11) **EP 2 046 190 B1**
(45) Veröffentlichungstag und Bekanntmachung des Hinweises auf die Patenterteilung: **07.04.2010**
(21) Anmeldenummer: 07765224.6
(22) Anmeldetag: 18.07.2007
(51) Int. Cl.: A61B 5/00

(54) **MESSUNG DER GLUKOSEKONZENTRATION IN PULSIERENDEM BLUT**
MEASURE OF GLUCOSE CONCENTRATION IN PULSATING BLOOD
MESURE DE LA CONCENTRATION EN GLUCOSE DANS LE SANG PULSATIF

(30) Priorität: 04.08.2006 DE 102006036920
(43) Veröffentlichungstag der Anmeldung: 15.04.2009
(73) Patentinhaber: Nirlus Engineering AG, 23560 Lübeck (DE)
(72) Erfinder: HERRMANN, Vera, 23560 Lübeck (DE)
(74) Vertreter: von dem Borne, Andreas
(86) Internationale Anmeldenummer: PCT/EP2007/006362
(87) Internationale Veröffentlichungsnummer: WO 2008/014890

(56) Entgegenhaltungen:
- WO-A-94/27495
- WO-A-98/19592
- US-A- 5 222 496
- US-A- 5 553 613
- US-A1- 2006 009 727

## Beschreibung

Die Erfindung betrifft ein Verfahren zur kontaktlosen Blutzuckermessung mittels NIR ("near infrared") Spektrometrie in fließendem, pulsierendem Blut, welches insbesondere einem lebenden Organismus zunächst entnommen und nach einer Behandlung, z.B. Dialyse, wieder zugeführt wird. Die Erfindung betrifft auch eine Vorrichtung, die als zusätzlicher oder integraler Bestandteil eines Dialyse-Gerätes zum Monitoring des Blutzuckergehalts geeignet ist. Die Erfindung ist überdies anwendbar in der nicht-invasiven in vivo Blutzuckerspiegel-Überwachung.

Die Bestimmung des Blutzuckerspiegels ohne direkte Kontaktierung des Blutes, insbesondere ohne eine für diesen Zweck gesonderte Blutentnahme, ist seit mehr als zwei Jahrzehnten Gegenstand intensiver medizinischer Forschung und Entwicklung. Hauptzielsetzung ist dabei das Bereitstellen eines kompakten, portablen Messgerätes für Diabetiker, dass idealerweise maximal durch Hautkontakt und ohne Verletzung der Haut schnell verlässliche Zuckerwerte liefern kann. Trotz erheblicher Anstrengungen zahlreicher Forscher, aus denen eine Vielzahl interessanter Lösungsansätze hervorging, hat bis heute kein zufrieden stellendes Messgerät dieser Art die Marktreife erreicht.

US 5,553,613 offenbart eine Vorrichtung und ein Verfahren zur fortlaufenden Messung der Glukosekonzentration in pulsierenden fließendem Blut, mit den Verfahrensschritten:
- Bestimmen eines Wertes für die Glukosekonzentration für einen Messzyklus, wobei
   - innerhalb des Messzyklus das Transmissions- oder Streuvermögens des Blutes für wenigstens zwei eingestrahlte NIR-Wellenlängen mehrfach erfasst wird,
   - ein von der Blutglukosekonzentration abhängiger Indikatorwert berechnet wird und
   - die Blutglukosekonzentration durch Vergleich des Indikatorwertes mit einer zuvor bestimmten Kalibriertabelle ermittelt wird;
- Bestimmen der Bluttemperatur während des Erfassens des Transmissions- oder Streuvermögens;
- fortlaufendes Messen der Pulsdauer des pulsierenden Blutflusses, wobei die Dauer des Messzyklus schritthaltend als ganzzahliges Vielfaches der Pulsdauer eingerichtet ist.

Der Stand der Technik, der an dieser Stelle nur auszugsweise gewürdigt werden kann, befasst sich sowohl mit der in vivo als auch der in vitro Messung, wobei sehr oft aus experimentellen in vitro Ergebnissen unmittelbar auf den in vivo Fall übertragen wird. Eine solche Übertragung ist allerdings prinzipiell nicht haltbar, da sie die erheblichen Komplikationen der Wechselwirkungen aller Blutbestandteile und des festen Gewebes mit Licht nicht oder nur unvollständig in Betracht zieht.

So ist beispielsweise der gelegentlich gemachte Vorschlag der Analyse des vom lebenden Körper zurück gestreuten NIR-Lichts in Wahrheit schon für sich genommen eine eigene Problemklasse, bei der die Aussagekraft des gestreuten Lichts zunächst in Frage steht. Da gestreute Photonen einen nicht-linearen, durch Vielfachstreuung beeinflussten Weg zurück an die Körperoberfläche nehmen, gilt es am Detektor zu entscheiden, welcher Lichtanteil überhaupt ein Blutgefäß durchlaufen hat und somit eine Information über den Blutzuckerspiegel tragen kann. Allein eine solche Quellenlokalisierung ist technisch aufwendig und z.B. in der DE 103 11 408 B3 beschrieben.

Manche Quellen widmen sich deshalb in erster Linie der Frage nach der physikalischen Messgröße, die zur Glukosemessung herangezogen werden soll. Die messtechnischen Feinheiten, die eine in vivo Messung tatsächlich erfordern würde, werden demgegenüber schlicht als fachmännisch lösbar angenommen.

So schlägt beispielsweise die US 5,009,230 vor, die Änderung der Polarisation von linear polarisiertem IR-Licht beim Durchleuchten von durchblutetem Gewebe zu messen, konkret die Drehung der Polarisationsebene durch Glukose-Moleküle. Die messbare Lichtintensität hinter einem Polarisationsfilter wird zur Konzentrationsbestimmung herangezogen. Dabei wird für die Sensitivität als wichtig erachtet, periodisch zwischen zueinander senkrechten Polarisationsrichtungen zu wechseln.

Aus der US 5,222,496 ist bekannt, dass die Intensität transmittierten oder reflektierten NIR-Lichts für mehrere Wellenlängen zueinander ins Verhältnis zu setzen ist, um die Glukosekonzentration zu messen. Insbesondere wird Licht um 1600 nm Wellenlänge benutzt, das infolge von Molekülschwingungen besonders gut von Glukose absorbiert wird. Wasser weist für diesen Wellenlängenbereich hingegen ein lokales Absorptionsminimum auf. Um die Signale anderer Blutbestandteile, aber auch den Einfluss des umliegenden Gewebes oder etwa der Hautpigmentierung bei der in vivo Messung zu kompensieren, schlägt die US 5 222 496 die zusätzliche Verwendung wenigstens einer weiteren Wellenlänge in der Nähe der ersten vor, die wiederum nicht von Glukose absorbiert werden soll. Es wird besonderer Wert auf den geringen Unterschied der Wellenlängen - weniger als 300 nm, vorzugsweise 60 nm - gelegt, um gleichartiges Streuverhalten sicherzustellen.

Beide Quellen kümmern sich indes überhaupt nicht um etwa die Bewegung des Blutes im lebenden Organismus. Auch die zweifellos zur spektrometrischen Analyse erforderliche Kenntnis der Temperatur des Blutes wird von der US 5,009,230 nur kurz angedeutet, aber in keiner Weise behandelt.

Es ist vermutlich der offensichtlich immensen Komplexität der Messaufgabe geschuldet, dass auch immer wieder indirekte Methoden zur Bestimmung von Blutzukker vorgeschlagen werden. Exemplarisch sei hier auf die photoakustische Messung verwiesen, bei der lebendiges Gewebe mit verschiedenen Wellenlängen bestrahlt wird, um die Wärmeexpansion während der Absorption der Strahlung in Form von detektierbaren Ultraschallwellen an der Hautoberfläche zu erfassen, siehe z.B. US 6,484,044 B1. Auch hierbei werden die Wellenlängen nach den bekannten Absorptionsmaxima von Glukose ausgewählt, und es finden ebenfalls differentielle Messungen zum Zwecke der Signalisolation statt.

Das prinzipielle Problem der Komplexität ist damit jedoch keinesfalls umgangen, geschweige denn gelöst. Wie schon bei der Rückstreuung von Photonen ist auch hier der Informationsgehalt der Schallsignale ungewiss, ihre genaue Quellenlokalisierung unklar und ihr Zustandekommen sicherlich von zahlreichen höchst individuellen und womöglich sogar veränderlichen Gewebeparametern beeinflusst. Bei der photoakustischen Methode handelt es sich letzten Endes um ein in höchstem Maße empirisches Verfahren, das sich offenbar mit dem Auffinden einer Standard-Kalibrierung für die breite Anwendbarkeit sehr schwer tut.

Abschließend sei als Stand der Technik noch das Verfahren der Gluco Watch® genannt, welches als bislang einziges mit einer Zulassung der FDA am Markt präsent ist. Die Gluco Watch® benötigt in der Tat keine Blutprobe zur Analyse, wird aber auf der Haut des Tragenden so befestigt, dass sie Fluid durch die Haut aufnehmen kann. Es wird des Öfteren von Hautirritationen bei Anwendern berichtet, und überdies wird vom Hersteller selbst abgeraten, die Gluco Watch® als einziges Mittel zur Beurteilung der richtigen Insulin-Dosierung zu verwenden.

Nach Ansicht der Anmelderin wird ein Verfahren zur nicht-invasiven Blutglukosemessung generell nicht auf empirische Dateninterpretation verzichten können. Doch sollte diese im Sinne der möglichst universellen Einsatzfähigkeit eines solchen Systems auf gut kontrollierbare Teilbereiche des Verfahrens beschränkt bleiben.

Zur Schaffung eines nicht-invasiven Systems sind folgende Aufgaben zu lösen:
1. Blutzuckermessung in fließendem, pulsierendem Blut (in vitro) unter Aufnahme empirischer, weitgehend universeller Kalibrierkurven,
2. Übertragung des Verfahrens vom in vitro Aufbau auf vorzugsweise große Blutgefäße (z.B. Aorta) durch
   a) Quellenlokalisierung, Elimination von Signalen ohne Information,
   b) Kompensation von Gewebe- und Hauteinflüssen auf das verbleibende Signal,
   c) In situ Temperaturbestimmung in Blutgefäß und Zwischengewebe zur Anwendung der Kalibrierkurven.

Zu den Punkten 2 a) und b) wurden bereits wesentliche Vorarbeiten von der Anmelderin in der DE 103 11 408 B3 publiziert. Die Aufgabe 2 c) ist Gegenstand zukünftiger Arbeit und wird zu gegebener Zeit in einer eigenen Anmeldung dargelegt werden. Die vorliegende Anmeldung befasst sich allein mit der Aufgabe 1. Die US 5,222,496 wird hier als nächstkommender Stand der Technik aufgefasst.

Die in vitro Bestimmung der Blutglukosekonzentration ist für sich genommen von Interesse zur Integration in Dialyse-Geräte. Untersuchungen in den USA zeigen die Wichtigkeit der kontinuierlichen Überwachung vor allem bei Dialyse-Patienten mit Diabetes. In Ermangelung geeigneter Vorrichtungen sind bereits Todesfälle zu beklagen gewesen.

Die Aufgabe der Erfindung besteht also darin, ein Verfahren und eine Vorrichtung zur kontaktlosen Messung der Blutglukosekonzentration in fließendem, pulsierendem Blut bereitzustellen.

Die Aufgabe wird gelöst durch das Verfahren mit den Merkmalen des Hauptanspruchs. Die Unteransprüche geben vorteilhafte Ausgestaltungen sowie eine Vorrichtung zur Durchführung des Verfahrens an.

Der nachfolgend geschilderten Erfindung liegt die Erkenntnis zugrunde, dass das Streuverhalten des fließenden, pulsierenden Blutes für NIR-Licht (Messlicht) einen dominanten Einfluss auf transmittierte und/oder gestreute Messlichtintensitäten besitzt.

Bereits die Lichtstreuung in einer ruhenden Blutprobe ist stark von den darin vorhandenen Blutsubstanzen (etwa Lipid, Alkohol etc.), aber auch von Anzahl und Form der im Blutplasma vorhandenen Streuteilchen - insbesondere rote und weiße Blutkörperchen, die nicht sphärische Form besitzen - geprägt. Selbst in ruhendem Blut bewegen sich diese Teilchen gegeneinander, verdrehen ihre relative Lage und bewirken eine fortwährende Änderung des richtungsabhängigen Streuvermögens. Die Eigenbewegung der Streupartikel hängt mit der Temperatur der Blutprobe zusammen. Überdies ist auch noch das NIR-Absorptionsvermögen von Wasser von der Temperatur abhängig.

Es ist daher ein erstes Merkmal der Erfindung, die Bluttemperatur durch eine separate Messung zu bestimmen, wobei wenigstens eine Genauigkeit von 0,5°C, vorzugsweise sogar 0,1 °C oder besser erreicht werden muss.

Um die Intensität des transmittierten oder rückgestreuten Messlichtes gegenüber der partikelbedingten Streuung unempfindlich zu machen, wurde gefunden, dass eine statistische Mittelung der Messwerte über eine Mehrzahl aufeinander folgender Zeitfenster erfolgen muss. Dabei sollten die Zeitfenster vorzugsweise jeweils wenige Millisekunden, maximal aber 100 ms, lang sein und in ihrer Gesamtheit (umfassend eine Sequenz nicht überlappender Zeitfenster, i. F. als Messzyklus bezeichnet) eine Zeitspanne von wenigstens einer Sekunde, vorzugsweise 2-3 Sekunden, überdecken. Damit stehen für die Auswertung wenigstens 10, vorzugsweise aber 200 oder mehr, diskrete Zeitfenster zur Verfügung.

Innerhalb eines jeden Zeitfensters sind überdies jeweils wenigstens 10.000 diskrete Messwerte zu erfassen, vorzugsweise sogar eher 30.000. Die Messwerte innerhalb der Zeitfenster erfassen also Intensitätsschwankungen auf der Mikrosekunden-Skala. Diese Skala ist für die Bluteigenbewegung nicht relevant, d.h. das Blut ist quasistatisch. Sie wird vielmehr zur Separation der Lichtsignale verwendet (s. u.).

Erfindungsgemäß werden die pro Zeitfenster aufgezeichneten, wenigstens 10.000 Messwerte von einem Prozessrechner (z:B. PC mit Datenerfassungskarte) aufgezeichnet und in einem Array abgelegt. Die Messwerte des nachfolgenden Zeitfensters werden in gleicher Anzahl erfasst und demselben Array hinzuaddiert. Die gespeicherten Messwerte wachsen somit kumulativ mit jedem weiteren Zeitfenster bis der Messzyklus endet (wenigstens 10 Zeitfenster, Mindestmessdauer 1 Sekunde). Abschließend kann das kumulierte Messwert-Array durch die Zahl der beitragenden Zeitfenster dividiert werden zur Normierung. Eine Normierung ist jedoch nicht zwingend erforderlich, da im Weiteren mit Messverhältnissen gearbeitet wird.

Für eine ruhende Blutprobe reicht dieses Vorgehen aus, die Einflüsse der Eigenbewegung von Streupartikeln im Blut durch Mittelung zu eliminieren. Für die in einem einzelnen Zeitfenster erfassten Messwerte wird so eine Ensemblemittelung über alle Bewegungszustände des Blutes vorgenommen. Gleichwohl ist das Ausmaß der Partikelbewegung im Blutplasma bestimmend für die effektive mittlere Streustärke der Probe. Die Streustärkenvariabilität wird durch die Ensemblemittelung nivelliert, aber nicht absolut erfasst. Schon deshalb ist zusätzlich die Temperaturmessung erforderlich.

Wenn das Blut nun während der Messung pulsierend fließt, treten weitere Bewegungszustände des Blutes auf, die sich im Wesentlichen periodisch mit der Pulsfrequenz wiederholen. Das Blut ist insbesondere Druckschwankungen ausgesetzt und wird zusätzliche Verwirbelungen und Dichteunterschiede aufweisen. Es ist daher erfindungsgemäß vorgesehen, die Ensemblemittelung - und damit die Länge des Messzyklus - über wenigstens einen vollständigen Pulsverlauf auszudehnen. Die Pulsfrequenz beträgt bei einem gesunden Erwachsenen im Wachzustand ungefähr 1 Hz, so dass dies innerhalb der oben genannten Spezifikation des Messzyklus von 2 bis 3 Sekunden problemlos möglich ist. Es ist erfindungsgemäß vorgesehen, den Messzyklus möglichst genau auf ein ganzzahliges Vielfaches der Pulsdauer einzurichten, um jeden wiederkehrenden Bewegungszustand des Blutes mit gleichem Gewicht in der Ensemblemittelung zu berücksichtigen.

Dabei kann die Pulsfrequenz einerseits separat durch Sensoren erfasst und dem Prozessrechner mitgeteilt werden, so dass dieser Zahl und Länge der einzelnen Zeitfenster fortlaufend neu berechnet und die Datenerfassungseinheit entsprechend ansteuert. Es ist aber generell sogar möglich, aus den aufgezeichneten Messwerten direkt auf die Pulsfrequenz zu schließen. Hierfür wird die oben skizzierte Ensemblemittelung zunächst unter Vorgabe einer hypothetischen Pulsfrequenz vorgenommen und dann unter Variation der Pulsfrequenz als Fitparameter optimiert.

Als Kenngröße für die Optimierung kann beispielsweise das integrierte Messsignal über das einzelne Zeitfenster nach Ensemblemittelung (die wenigstens 10.000 kumulierten Messwerte) dienen, welches ein Maß für die Gesamtstreustärke des Blutes ist. Letztere ist zwar temperaturabhängig, doch über den kurzen Zeitraum einiger aufeinander folgender Messzyklen (einige Male 2-3 Sekunden) kann die Temperatur als praktisch konstant vorausgesetzt werden. Ist das Ensemble von Blutbewegungszuständen im Hinblick auf die Pulsdauer ungünstig gewählt, wird die Kenngröße zwischen aufeinander folgenden Messungen deutliche Variationen zeigen. Das Optimalitätskriterium ist hier die Minimierung dieser Variationen.

Es lassen sich gewiss andere Mess- und/oder Berechnungsverfahren für die Pulsdauer finden und einsetzen. Erfindungsgemäß ist hier wichtig, die Pulsdauer bei der Festlegung der Zeitfenster und des Messzyklus für die statistische Auswertung zu berücksichtigen. Es kann im Übrigen vorteilhaft sein, mit fest gewählten Zeitfenstern von maximal 100 ms zu arbeiten, so dass es nicht möglich ist, den gesamten Puls exakt mit einer ganzen Anzahl von Zeitfenstern abzutasten. In diesem Fall empfiehlt sich die Einführung einer Messtotzeit, i. a. kleiner als das feste Zeitfenster, um die gewünschte Synchronität mit dem Puls herzustellen. Mit Messtotzeit ist gemeint, dass Messwerte der Lichtdetektoren während dieser Zeit nicht beachtet oder gar nicht erzeugt werden. Die Messtotzeit kann wie oben beschrieben auch dynamisch optimiert werden.

Vorzugsweise wird man den Messzyklus über eine Mehrzahl von Pulsschlägen ausdehnen. Diese Mehrzahl wird jedoch typisch eine kleine Zahl sein (< 10), da ein Messwert binnen weniger Sekunden vorliegen muss. Dies ist besonders wichtig für ein in vivo System, bei dem der Nutzer selbst die Messung vornehmen soll. Längere Messdauem gäben Anlass zu Bewegungsartefakten.

Zur spektrometrischen Bestimmung der Blutglukosekonzentration wird nun teilweise der US 5 222 496 gefolgt, indem eine NIR-Wellenlänge aus dem Bereich 1560-1630 nm, vorzugsweise 1600 nm in das Blut eingestrahlt wird. Grundsätzlich können Transmission- und/oder Streuvermögen des Blutes für die gewählten Wellenlängen gemessen und als Indikator für die Blutglukosekonzentration herangezogen werden.

Bei der in vitro Messung wird bevorzugt die Transmission des eingestrahlten Lichts aufgezeichnet, um den Extinktionskoeffizienten (auch: optische Dichte) zu messen. Dieser ist definiert als negativer dekadischer Logarithmus des Verhältnisses der transmittierten zur eingestrahlten Lichtintensität. Er wird aus den wenigstens 10.000 Messwerten des ermittelten Zeitfensters nach der Ensemblemittelung berechnet.

Bei der Bestimmung des Extinktionskoeffizienten ist zu beachten, dass der IR-Lichtdetektor auch ungewollte Lichtanteile erfassen kann, die die Daten verfälschen. Zwar ist es möglich, verschiedene Detektoren mit unterschiedlicher chromatischer Sensitivität zu verwenden, doch auch diese registrieren Fremdlicht in ihrem jeweiligen sensitiven Wellenlängenbereich. Deshalb wird das eingestrahlte Licht vorzugsweise amplitudenmoduliert mit einer Modulationsfrequenz von wenigstens 1 MHz, besonders bevorzugt 3-4 MHz. Diese Amplitudenmodulation ist in den wenigstens 10.000 Messwerten innerhalb des maximal 100 ms andauernden Zeitfensters prinzipiell auflösbar und erlaubt die Spektralanalyse der Daten im Zeitfenster. Vorzugsweise mittels Fast Fourier-Transformation wird eine Spektraldarstellung der Messwerte des Zeitfensters nach der Ensemblemittelung berechnet. In die weitere Auswertung gehen dann nur noch Fourierkomponenten aus dem Bereich der Modulationsfrequenz ein. Dabei kann im einfachsten Fall die Fourierkomponente zur Modulationsfrequenz allein ggf. durch Interpolation bestimmt und als Maß für die transmittierte Intensität angenommen werden. Es hat sich jedoch als vorteilhaft erwiesen, eher ein numerisches Integral über das Fourierspektrum im Frequenzraum zu verwenden, wobei Werte in einem Fenster der Breite 2 Δf aufsummiert werden. Dabei liegt die Modulationsfrequenz mittig im Integrationsfenster. Δf sollte möglichst klein gewählt werden, aber ausreichend groß, um Fluktuationen zwischen aufeinander folgenden Messungen (im Abstand einiger Sekunden), zwischen denen sich die zu messende physikalische Größe nicht wesentlich geändert haben kann, zu kompensieren. Es handelt sich also um einen Fitparameter, der automatisch während der Messung variiert und angepasst werden kann. Vorzugsweise wird er irgendwann in der Auswerteeinheit als Konstante gespeichert und für spätere Messungen wieder verwendet. Er kann natürlich gelegentlich überprüft und neu eingerichtet werden.

Unmodulierte Fremdlichtanteile sind nach dem zuvor Gesagten praktisch eliminiert. Die eingestrahlte Intensität wiederum skaliert mit der Laserleistung und ist bekannt. Damit kann der Extinktionskoeffizient wie oben definiert angegeben werden.

Der Extinktionskoeffizient E hängt wie oben beschrieben von den Bewegungszuständen des Blutes ab, aber eben auch von der schieren Zusammensetzung der Blutpartikel insbesondere hinsichtlich Art und Anzahl, kurz von den Hämatokritwerten, die sich zwischen verschiedenen Personen wesentlich unterscheiden können. Überdies spielt der Fettpegel im Blut eine Rolle, der sogar stundenweise in derselben Person variieren kann.

Zur Kompensation wird deshalb zeitgleich eine zweite NIR-Wellenlänge eingestrahlt, deren Transmission nur von Hämatokritwerten und Fettgehalt, nicht aber von anderen Faktoren wie Blutzucker oder z.B. der Sauerstoffsättigung des Blutes abhängt. Hierfür bietet sich die "isobestische" Wellenlänge bei etwa 808 nm besonders an, für die die Absorptionsvermögen von Oxy- und Deoxyhämoglobin als identisch bekannt ist. Sie liegt zugleich im ausgedehnten Absorptionsminimum von Wasser. Hierin unterscheidet sich die erfindungsgemäße Vorgehensweise bereits signifikant von der Lehre der US 5,222,496.

Es sind grundsätzlich Variationen der genannten Wellenlängen möglich, d.h. auch Werte in der Umgebung von 808 nm (voraussichtlich im Bereich 790-815 nm) können in Betracht gezogen werden. Da das Licht vorzugsweise aus Laserdioden eingestrahlt wird, kann es hier als technisch sehr vorteilhafte Ausgestaltung in Betracht kommen, anstelle zweier Laser nur einen einzigen mit einem frequenzverdoppelnden Medium und Strahlteiler zu verwenden. Dies kann dazu dienen, die eingestrahlten Intensitäten der unterschiedlichen Wellenlängen in ein festes, von Pumpleistung und Laseransteuerung prinzipiell unabhängiges Verhältnis zueinander zu setzen.

Für die isobestische Wellenlänge wird der Extinktionskoeffizient EISO gemessen. Das Verhältnis R=E / EISO wird im Prozessrechner bestimmt und der Blutzuckerwert kann anhand der im Rechner als Tabelle vorhandenen Kalibrierfunktion K (R, T) abgelesen werden. Dabei ist T die zugleich zu messende Bluttemperatur, die im einfachsten Fall bei der in vitro Messung mittels eines Temperaturfühlers zu erfassen ist. Es ist dabei nicht notwendig, den Fühler mit dem Blut in direkten Kontakt zu bringen, sondern er kann außen an einer Messküvette installiert sein. Überdies besteht auch die Möglichkeit, die Temperatur über die vom Blut emittierte Infrarotstrahlung zu detektieren, wenn die NIR-Laserquellen zeitweise abgeschaltet werden (z.B. während der oben erwähnten Messtotzeit, wenigstens ein IR-Detektor ist ja ohnehin vorhanden.

Die Kalibriertabelle K (R, T) ist einmal zu bestimmen, indem NIR-Messwerte mit Blutglukosedaten aus anderen Messverfahren, etwa mittels Messstreifen, bestimmt werden.

Abschließend soll ein konkretes Ausführungsbeispiel für ein System zur in vitro Blutzuckermessung angegeben sowie die Ergebnisse einiger Beispielmessungen vorgestellt werden. Dazu dienen die folgenden Figuren:
- Fig. 1: zeigt eine Aufbauskizze der Vorrichtung zur Durchführung des hier beschriebenen Verfahrens;
- Fig. 2: stellt die spektrometrisch gemessenen Blutzuckerwerte denen einer gleichzeitigen Messung mittel Blutzuckerteststreifen nach dem Stand der Technik gegenüber.

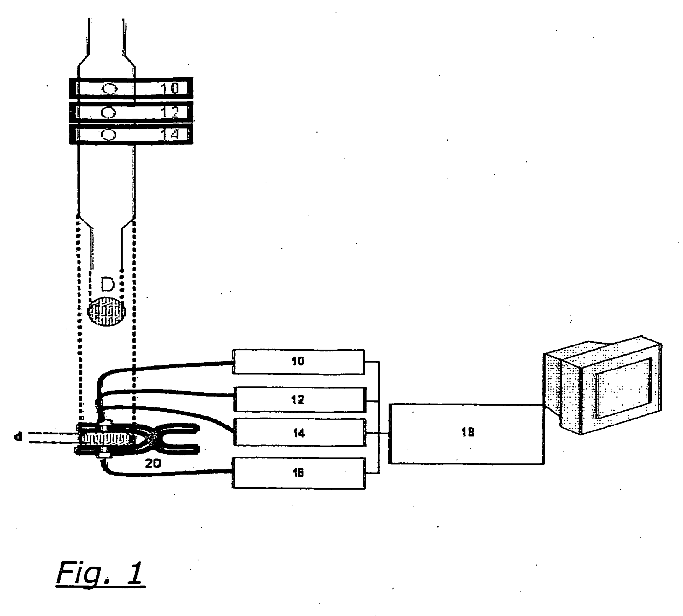

In Fig. 1 ist im oberen Bildbereich die Seitenansicht einer Messküvette dargestellt, wobei an einer Seite (hier dem Betrachter zugewandt) zwei Laserlichtquellen 10 und 14 sowie ein Temperaturfühler 12 angeordnet sind. Die Laserlichtquellen 10 und 14 sind vorzugsweise die Austrittsenden von Lichtleiterfasern, könnten aber auch Laserdioden sein, die vor Ort elektrisch versorgt werden. Im unten gezeigten Laborexperiment wurden durchstimmbare Laserquellen des Typs High Performance Tunable Laser TSL-510 SANTEC benutzt, was aber schon aus Kostengründen keine bevorzugte Ausgestaltung der Erfindung sein soll. Die Einstrahlleistung sollte in der hier beschriebenen Vorrichtung vorzugsweise bei 10 mW liegen.

Der Temperaturfühler 12 misst die Außentemperatur der Küvette. Diese kann ohne weiteres durch einmalige Kalibrierung zum Rückschluss auf die Bluttemperatur dienen. Jedoch ist darauf zu achten, dass die Messküvette mit Temperaturfühler 12 gegen Temperaturschwankungen sehr gut abgeschirmt ist. Bereits das Öffnen eines Fensters könnte sonst zu Fehlmessungen führen.

Eine Messküvette, die beispielsweise in die Zuleitungen eines Dialyse-Gerätes integriert wird, sollte aus einem für NIR-Licht durchlässigen Material (z.B. Quartzglas, Chalkogenid Infrarot (CIR) Glas) bestehen und wird typisch im Bereich der Verbindungen zu den Zuleitungen einen kreisförmigen Querschnitt von D = 4,2 -4,5 mm Durchmesser aufweisen (vgl. Fig. 1). Im dazwischen befindlichen Durchleuchtungsbereich muss der Durchmesser in Durchstrahlrichtung auf etwa d= 2 mm verringert werden. Fig. 1 unten zeigt eine Querschnittsdarstellung, wobei eine Klemmspange 20 dazu verwendet wird, die abgeflachte Messküvette zu halten. Ebenfalls dargestellt sind die Laserquellen 10 und 14 sowie der Temperaturfühler 12 auf der einen Seite der Messküvette. Auf der gegenüber liegenden Seite der Messküvette ist wenigstens ein NIR-Detektor 16 angeordnet, der die transmittierte Strahlung misst. Es wurde konkret ein NFI-2053-FC-M 10 MHz InGaAs-Photoreceiver eingesetzt. Bevorzugt wird ein NIR-Laser mit Strahlteiler und frequenzverdoppelndem Medium, der gleichzeitig NIR-Licht mit 1560-1630 nm Wellenlänge sowie NIR-Licht mit der halben Wellenlänge emittiert zur Darstellung der wenigstens zwei NIR-Lichtquellen.

Fig. 1 stellt schließlich noch die rechnergestützte Datenerfassungs- und Auswerteeinheit 18 dar sowie eine Anzeige der ermittelten Werte für die Blutglukosekonzentration. Hierbei eignen sich beispielsweise die Highspeed Datenerfassungskarte WA1-100-110 von Acquitec mit Abtastrate 20 MHz und Auflösung 12 Bit sowie die AcquiFlex Oszilloskop-, Waveform-Editor- und Logik-Analysator-Software. Die Vorrichtung verfährt wie zuvor beschrieben und erlaubt nun die kontinuierliche Überwachung des Blutes.

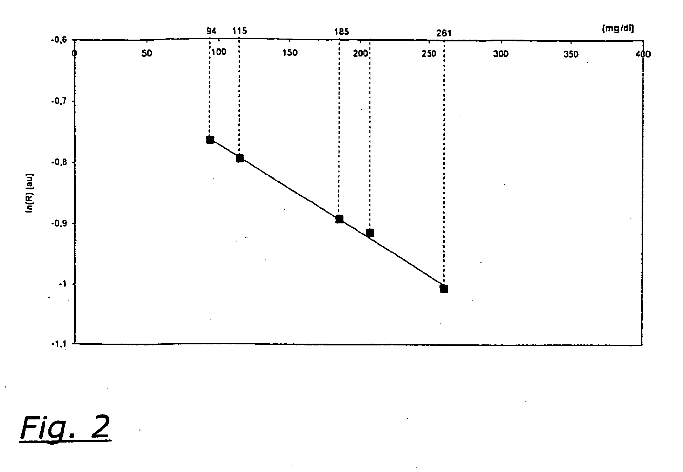

Fig. 2 belegt anhand einiger Beispielmessungen an Menschenblut, dass die zur Nutzung der erfindungsgemäßen Vorrichtung und des Verfahrens erforderliche Kalibrierung ermittelt werden kann. Dafür werden zunächst Referenzwerte (z.B. mit dem kommerziellen Accu-Chek ® Blutzucker-Messgerät, besser aber mit sehr viel genaueren Analyse-Verfahren) an Probanden bestimmt. Auf der Abszisse sind die "wahren" Blutzuckerwerte in Einheiten mg/dl aufgetragen, und die Ordinate zeigt den Logarithmus des Intensitätsverhältnisses R, wie es mit dem oben beschriebenen Verfahren an vom Arzt entnommenen Blutproben desselben Probanden bestimmt wird. Offenbar ordnen sich die "wahren" Werte - die allerdings selbst noch fehlerbehaftet sein können - etwa entlang einer Trendgerade an. Diese Trendgerade kann - bei bekannter Bluttemperatur - als Kalibrierkurve zur Übersetzung der spektrometrischen Werte benutzt werden.

In Fig. 2 sind nur vorläufige erste Ergebnisse zu sehen. Die Kalibrierkurven sind für eine Vielzahl von Bluttemperaturen und selbstverständlich mit einer sehr viel größeren Zahl von Blutproben zu ermitteln.

## Patentansprüche

1. Verfahren zur fortlaufenden Messung der Glukosekonzentration in pulsierend fließendem Blut mit den Schritten:
- Bestimmen eines Wertes für die Glukosekonzentration für einen ersten Messzyklus, und
- Wiederholen des Bestimmens dieses Wertes in darauf folgenden Messzyklen, wobei innerhalb jedes Messzyklus das Transmissions- und/oder Streuvermögens des Blutes für wenigstens zwei eingestrahlte NIR-Wellenlängen mehrfach erfasst, ein von der Blutglukosekonzentration abhängiger Indikatorwert berechnet und die Blutglukosekonzentration durch Vergleich des Indikatorwertes mit einer zuvor bestimmten Kalibriertabelle ermittelt wird,
- Bestimmen der Bluttemperatur während des Erfassens des Transmissions- und/oder Streuvermögens,
- fortlaufendes Messen der Pulsdauer des pulsierenden Blutflusses, wobei die Dauer jedes Messzyklus schritthaltend als ganzzahliges Vielfaches der Pulsdauer eingerichtet ist,
wobei die erste der wenigstens zwei NIR-Wellenlängen aus dem Wellenlängenbereich 1560-1630 nm ausgewählt wird, und die zweite der wenigstens zwei NIR-Wellenlängen aus dem Wellenlängenbereich 790-815 nm ausgewählt wird, und
das Verhältnis der Transmissions- und/oder Streuvermögen der wenigstens zwei Wellenlängen berechnet wird, wobei dieses Verhältnis in Relation zur Bluttemperatur als Indikatorwert zum Ablesen der Blutglukosekonzentration aus der Kalibriertabelle dient.

2. Verfahren nach Anspruch 1, **dadurch gekennzeichnet, dass** die wenigstens zwei NIR-Wellenlängen amplitudenmoduliert eingestrahlt werden mit Modulationsfrequenzen oberhalb von 1 MHz.

3. Verfahren nach Anspruch 1 oder 2, **dadurch gekennzeichnet, dass** der Messzyklus eine Mehrzahl nicht überlappender, gleich langer Zeitfenster umfasst, in denen jeweils dieselbe Anzahl von Messwerten für Transmissions- und/oder Streuvermögen des Blutes erfasst werd, wobei eine Ensemblemittelung über alle Zeitfenster des Messzyklus stattfindet, um am Ende des Messzyklus ein ermitteltes Zeitfenster enthaltend besagte Anzahl von mittleren Messwerten errechnet zu haben.

4. Verfahren nach Anspruch 3, **dadurch gekennzeichnet, dass** ein Zeitfenster weniger als 100 Millisekunden umfasst.

5. Verfahren nach Anspruch 3 oder 4, **dadurch gekennzeichnet**, die Anzahl der Messwerten für Transmissions- und/oder Streuvermögen des Blutes in einem Zeitfenster wenigstens 10.000 beträgt.

6. Verfahren nach einem der Ansprüche 3 bis 5, **dadurch gekennzeichnet, dass** die mittleren Messwerte im ermittelten Zeitfenster einer Fouriertransformation in den Frequenzbereich unterzogen werden.

7. Verfahren nach den Ansprüchen 2 und 6, **dadurch gekennzeichnet, dass** die Fourierkomponente der mittleren Messwerte zur Modulationsfrequenz der Amplitudenmodulation der eingestrahlten NIR-Wellenlängen als Maß für das Transmissions- und/oder Streuvermögen des Blutes ausgewertet wird.

8. Verfahren nach den Ansprüchen 2 und 6, **dadurch gekennzeichnet, dass** ein Integral im Frequenzraum über die Fourierkomponenten der mittleren Messwerte in einem Intervall um die Modulationsfrequenz der Amplitudenmodulation der eingestrahlten NIR-Wellenlängen als Maß für das Transmissions- und/oder Streuvermögen des Blutes ausgewertet wird.

9. Verfahren nach einem der vorangehenden Ansprüche, **dadurch gekennzeichnet, dass** die schritthaltende Anpassung der Dauer eines Messzyklus an die fortlaufend erfasste Pulsdauer des Blutes unter Verwendung einer variablen Anzahl von Zeitfenstern mit zuvor festgelegter Dauer und unter Hinzufügen einer variablen Messtotzeit erfolgt, wobei die Anzahl der Zeitfenster und die Messtotzeit schritthaltend berechnet werden.

10. Vorrichtung zum Monitoring der Blutzuckerkonzentration nach einem der Verfahren der Ansprüche 1 bis 9, wobei das Blut einem lebenden Organismus in einem Kreislauf entnommen, gemessen und wieder zugeführt wird, mit
- einer Messküvette ausgebildet für den kontinuierlichen Zu- und Abfluss von Blut mit einem abgeflacht ausgebildeten Durchleuchtungsbereich,
- wenigstens zwei NIR-Lichtquellen angeordnet an einer Flachseite der Messküvette zur Durchleuchtung der Messküvette in Richtung auf die gegenüberliegende Flachseite,
- wenigstens einem NIR-Detektor angeordnet auf der Flachseite, die jener mit den NIR-Lichtquellen gegenüberliegt,
- einer Vorrichtung zum Messen der Bluttemperatur wenigstens im Durchleuchtungsbereich der Messküvette,
- einer Auswerteeinheit zur Ermittlung der Werte für die Blutglukosekonzentration.

11. Vorrichtung nach Anspruch 10, **gekennzeichnet durch** einen NIR-Laser mit Strahlteiler und frequenzverdoppelndem Medium, der gleichzeitig NIR-Licht mit 1560-1630 nm Wellenlänge sowie NIR-Licht mit der halben Wellenlänge emittiert zur Darstellung der wenigstens zwei NIR-Lichtquellen.

## Claims

1. A method for the continuous measurement of the glucose concentration in pulsating, flowing blood with the steps:
- determination of a value for the glucose concentration for a first measurement cycle, and
- repetition of the determination of this value in subsequent measurement cycles, wherein within each measurement cycle the transmission and/or scattering properties of the blood are multiply recorded for at least two irradiated NIR wavelengths, an indicator value dependent on the blood glucose concentration is calculated, and the blood glucose concentration is determined by comparison of the indicator value with a previously determined calibration table,
- determination of the blood temperature during the recording of the transmission and/or scattering properties,
- continuous measurement of the pulse duration of the pulsating blood flow, wherein the duration of each measurement cycle is adjusted on-line as a whole number multiple of the pulse duration, wherein
the first of the at least two NIR wavelengths is selected from the wavelength range of 1560-1630 nm, and the second of the at least two NIR wavelengths is selected from the wavelength range of 790-815 nm, and
the ratio of the transmission and/or scattering properties of the at least two wavelengths is calculated, wherein this ratio in relation to the blood temperature serves as an indicator value for the read-out of the blood glucose concentration from the calibration table.

2. The method according to Claim 1, **characterised in that** the at least two NIR wavelengths are irradiated with modulated amplitudes, with modulation frequencies above 1 MHz.

3. The method according to Claim 1 or 2, **characterised in that** the measurement cycle comprises a multiplicity of non-overlapping time windows of equal length, in each of which the same number of measurement values for the transmission and/or scattering properties of the blood is recorded, wherein an ensemble averaging over all time windows of the measurement cycle takes place, so as to have computed at the end of the measurement cycle a time window as determined, containing a stated number of average measurement values.

4. The method according to Claim 3, **characterised in that** the time window comprises less than 100 milliseconds.

5. The method according to Claim 3 or 4, **characterised in that** the number of measured values for the transmission and/or scattering properties of the blood in one time window is at least 10,000.

6. The method according to one of the Claims 3 to 5, **characterised in that** the average measured values in the time window determined are subjected to a Fourier transformation in the frequency range.

7. The method according to the Claims 2 and 6, **characterised in that** the Fourier component of the average measured values for the modulation frequency of the amplitude modulation of the irradiated NIR wavelengths is evaluated as a measure for the transmission and/or scattering properties of the blood.

8. The method according to the Claims 2 and 6, **characterised in that** an integral in the frequency space over the Fourier components of the average measured values is evaluated in an interval around the modulation frequency of the amplitude modulation of the irradiated NIR wavelengths as a measure for the transmission and/or scattering properties of the blood

9. The method according to one of the previous claims, **characterised in that** the on-line adaptation of the duration of a measurement cycle to the continuously recorded pulse duration of the blood takes place with the use of a variable number of time windows with previously defined durations and with the addition of a variable measurement dead time, wherein the number of the time windows and the measurement dead time are calculated on-line.

10. A device for the monitoring of the blood sugar concentration according to one of the methods of the Claims 1 to 9, wherein the blood is extracted from, measured and re-supplied to a living organism in a circuit, with
- a measurement cell designed for the continuous flow of blood to and from the cell with a trans-illumination region of flattened design,
- at least two NIR light sources arranged on one flat side of the measurement cell for the trans-illumination of the measurement cell in a direction towards the opposite flat side,
- at least one NIR detector arranged on the flat side that lies opposite to that with the NIR light sources,
- a device for the measurement of the blood temperature at least in the trans-illumination region of the measurement cell,
- an evaluation unit for the determination of the values for the blood glucose concentration.

11. A device according to Claim 10, **characterised by** an NIR laser with a beam divider and a frequency-doubling medium, which simultaneously emits NIR light with a 1560-1630 nm wavelength, as well as NIR light with half the wavelength, for the representation of the at least two NIR light sources.

## Revendications

1. Procédé de mesure continue de la concentration en glucose dans du sang s'écoulant en pulsant, comportant dans les étapes suivantes :
- définition d'une valeur pour la concentration en glucose du sang pour un premier cycle de mesure et
- répétition de la définition de cette valeur dans les cycles de mesure suivants, l'aptitude à la transmission et/ou la diffusion du sang étant détectée plusieurs fois pendant chaque cycle de mesure pour au moins deux longueurs d'ondes NIR irradiées, une valeur indicatrice dépendant de la concentration en glucose du sang étant calculée et la concentration en glucose du sang étant déterminée par comparaison de la valeur indicatrice avec un tableau de calibrage défini préalablement,
- définition de la température du sang pendant la détection de l'aptitude à la transmission et/ou la diffusion du sang,
- mesure continue de la durée de pulsation du flux sanguin en pulsation, la durée de chaque cycle de mesure étant définie étape par étape sous forme de multiple entier de la durée de pulsation,
la première des au moins deux longueurs d'ondes NIR étant sélectionnée dans la plage de longueurs d'ondes de 1560 à 1630 nm et la deuxième des au moins deux longueurs d'ondes NIR étant sélectionnée dans la plage de longueurs d'ondes de 790 à 815 nm et
le rapport d'aptitude à la transmission et/ou la diffusion des au moins deux longueurs d'ondes NIR étant calculé, ce rapport servant, en relation avec la température du sang, à lire la concentration en glucose du sang dans le tableau de calibrage.

2. Procédé selon la revendication 1, **caractérisé en ce que** les au moins deux longueurs d'ondes NIR sont irradiées avec une amplitude modulée avec des fréquences de modulation supérieures à 1 MHz.

3. Procédé selon la revendication 1 ou 2, **caractérisé en ce que** le cycle de mesure comprend une pluralité de fenêtres temporelles de même longueur et ne se chevauchant pas dans lesquelles respectivement le même nombre de valeurs de mesure pour l'aptitude à la transmission et/ou la diffusion du sang sont mesurées, sachant qu'est calculée une moyenne d'ensemble sur toutes les fenêtres temporelles du cycle de mesure afin d'avoir calculé à la fin du cycle de mesure une fenêtre temporelle déterminée contenant ledit nombre de valeurs de mesure moyennes.

4. Procédé selon la revendication 3, **caractérisé en ce qu'**une fenêtre temporelle comporte moins de 100 millisecondes.

5. Procédé selon la revendication 3 ou 4, **caractérisé en ce que** le nombre de valeurs de mesure pour l'aptitude à la transmission et/ou la diffusion du sang dans une fenêtre temporelle s'élève à au moins 10 000.

6. Procédé selon une des revendications 3 à 5, **caractérisé en ce que** les valeurs de mesure moyennes de la fenêtre temporelle déterminée subissent une transformation Fourier dans la plage de fréquence.

7. Procédé selon les revendications 2 et 6, **caractérisé en ce que** le composant Fourier des valeurs de mesure moyennes pour la fréquence de modulation de la modulation d'amplitude des longueurs d'ondes NIR irradiées est évalué en tant qu'indicateur de l'aptitude à la transmission et/ou la diffusion du sang.

8. Procédé selon les revendications 2 et 6, **caractérisé en ce qu'**une intégrale de l'espace de fréquence est évaluée à l'aide des composants Fourier des valeurs de mesure moyennes dans un intervalle autour de la fréquence de modulation de la modulation d'amplitude des longueurs d'ondes NIR irradiées en tant qu'indicateur de l'aptitude à la transmission et/ou la diffusion du sang.

9. Procédé selon les revendications précédentes, **caractérisé en ce que** l'adaptation étape par étape de la durée d'un cycle de mesure à la durée de pulsation détectée en continu du sang se fait en utilisant un nombre variable de fenêtres temporelles à durée fixée préalablement et en intégrant un temps mort de mesure variable, le nombre de fenêtres temporelles et le temps mort de mesure étant calculés étape par étape.

10. Dispositif de contrôle de la concentration en glucose du sang selon un des procédés des revendications 1 à 9, dans lequel le sang est prélevé, mesuré et renvoyé en circuit dans un organisme vivant et comportant
- une cuvette de mesure conçue pour acheminer et évacuer en continu du sang et dotée d'une zone d'éclairage en transparence à conformation aplatie,
- au moins deux sources lumineuses NIR disposées sur une face plate de la cuvette de mesure pour éclairer la cuvette de mesure en transparence en direction de la face plate opposée,
- au moins un détecteur NIR disposé sur la face plate et qui fait face à celui comportant la source lumineuse NIR,
- un dispositif de mesure de la température du sang au moins dans la zone d'éclairage en transparence de la cuvette de mesure,
- une unité d'évaluation pour la détermination des valeurs pour la concentration en glucose du sang.

11. Dispositif selon la revendication 10, **caractérisé par** un laser NIR doté d'un séparateur de faisceaux et d'un moyen de doublement de fréquence et qui émet en même temps de la lumière NIR ayant une longueur d'ondes de 1560 à 1360 nm de même que de la lumière NIR ayant la moitié de cette longueur d'ondes pour représenter les au moins deux sources lumineuses NIR.
